# EUROPEAN PATENT APPLICATION

(11) **EP 3 808 411 A1**
(43) Date of publication of application: **21.04.2021**
(21) Application number: 19813374.6
(22) Date of filing: 16.10.2019
(51) Int. Cl.: A61Q 19/08, A61K 8/365

(54) **COSMETIC COMPOSITIONS AND USE THEREOF**

(71) Applicant: Mesoestetic Pharma Group, S.L., 08840 Viladecans (Barcelona) (ES)
(72) Inventor: MARTÍNEZ GUTIÉRREZ, Alfredo, 08840 Viladecans (BARCELONA) (ES)
(74) Representative: Capitán García, Maria Nuria
(86) International application number: PCT/ES2019/070704
(87) International publication number: WO 2020/240056

(57) **Abstract**

The invention provides cosmetic compositions that make it possible to combat the effects of ageing on skin due to the process of cellular senescence, for example induced by Ultraviolet Type-B (UVB) damage, such as the appearance of wrinkles, loss of density, appearance of hyperpigmentation or loss of firmness, due to including, as a protective component against the process of cellular senescence, shikimic acid, acting through the activation of sirtuin 1 (Sirt1).

## Description

The present invention relates to cosmetic compositions, as well as the topical use thereof to combat the effects of ageing on skin.

More specifically, the invention provides cosmetic compositions that make it possible to combat the effects of ageing on skin due to the process of cellular senescence, for example induced by Ultraviolet Type-B (UVB) damage, such as the appearance of wrinkles, loss of density, appearance of hyperpigmentation or loss of firmness, due to including, as a protective component against the process of cellular senescence, shikimic acid, acting through the activation of sirtuin 1 (Sirt1).

Shikimic acid, or 3,4,5-trihydroxy-1-cyclohexene-1-carboxylic acid, better known for its anionic form shikimate, whose formula is shown below, is present in many plants and has various interesting biological properties. In addition to its pharmacological importance in itself, it is also used as an intermediate in the synthesis of many drugs, for example the antiviral agent oseltamivir.

It is also a key hydroaromatic intermediate in the biosynthetic pathway of the essential aromatic amino acid shikimate (L-phenylalanine, L-tyrosine and L-tryptophan), lignin and most of the alkaloids present in plants and microorganisms (Bochkov, DV; Sysolyatin SV; Kalashnikov, Al; Surmacheva, IA Shikimic acid: review of its analytical, isolation, and purification techniques from plant and microbial sources. J. Chem. Biol., 2012, 5, 5-17).

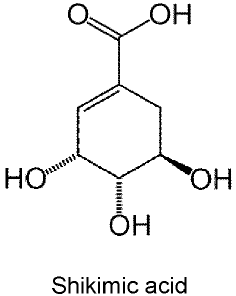

Since the shikimate pathway is not present in mammals, there is great potential for the design and synthesis of enzyme inhibitors that can selectively block transformations catalysed by specific enzymes in this pathway. In this context, different studies have been carried out on the design, synthesis and evaluation of antibacterial, herbicidal and antifungal agents with low environmental impact that may interfere with the specific transformations of this pathway without negative effects for mammals (Jiang, S. et al., "Synthesis of 3-deoxy-3,3-difluoroshikimic acid and its 4-epimer from quinic acid", Tetrahedron Asym., 1999, 10, 4087-4090; Song, C. et al. "Syntheses of (6S)-6-fluoro- and (6R)-6-hydroxyshikimic acids", Tetrahedron Lett., 2001, 42, 9069-9071).

Its biological properties include its activity as an antioxidant, anticoagulant and antithrombotic, antibacterial, anti-inflammatory and analgesic agent. In this context, its activity against DPPH and nitric oxide radicals has been reported in a concentration-dependent form (Oliveira, AP et al. Ficus carica L.: Metabolic and biological screening, Food Chem. Toxicol,. 2009, 47, 2841-2846; Vrchovská, V. et al. Assessing the antioxidative properties and chemical composition of Linaria vulgaris infusion, Nat. Prod. Res., 2008, 22, 735-746).

By way of example, shikimic acid can influence bile control (Okada, S.; Hara, K. Shikimic acid, a useful intermediate for manufacturing herbicides and agrochemicals and antibiotics, Japanese patent JP 61018720, January 27, 1986). Its use has also been described in the preparation of drugs for treating ulcerative colitis (Xing, J.; Sun, J.; Dong, Y.; Qu, Q.; Yuan, Z.; Hu, S.; Shi, R. Use of shikimic acid in preparing medicament for treating ulcerative colitis, Chinese patent CN 101352427, January 28, 2009).

Similarly, shikimic acid protects against oxidative stress in cells of the human nervous system and in human hepatocytes, in this case through the inhibition of NF-kB and the activation of Nrf2 (J Ethnopharmacol. 2014 Aug 8;155(1):132-46. doi: 10.1016/j.jep.2014.04.046. Epub 2014 May 14, "Protective effect of coconut water concentrate and its active component shikimic acid against hydroperoxide mediated oxidative stress through suppression of NF-κB and activation of Nrf2 pathway", Manna K *et al.)* and inhibits the destruction of extracellular matrix in human chondrocytes (Intlmmunopharmacol. 2018 Oct.; 63:155-160. doi: 10.1016/j.intimp.2018.07.021. Epub 2018 Aug 7, "Shikimic acid prevents cartilage matrix destruction in human chondrocytes", Guo X. *et al.*), it inhibits lipogenesis through phosphorylation of AMPK in human hepatocytes (Int J Mol Sci. 2019 Jan 29; 20 (3). pii: E582. doi: 10.3390/ijms20030582, "Hypolipogenic Effect of Shikimic Acid Via Inhibition of MID1IP1 and Phosphorylation of AMPK/ACC", Kim MJ. *et al*.), and inhibits the enzyme tyrosinase, involved in the synthesis of melanin in skin (EurRev Med Pharmacol Sci. 2016; 20(6):1214-20, "Skin whitening capability of shikimic acid pathway compound", Chen YH, *et al*.).

Thus, for example, document CN1113646 describes the use of shikimic acid for manufacturing an antithrombotic and analgesic drug. US patent application US5164382A describes the use of certain fluorinated derivatives of shikimic acid having antibacterial and antifungal effects.

In the field of cosmetic compositions, US20060216251 describes a topical composition comprising a lipoic acid or a dermatologically acceptable salt thereof, a carnitine or dermatologically acceptable salt thereof, a carnosine or dermatologically acceptable salt thereof and a dermatologically acceptable carrier, which may also include an antioxidant selected, inter alia, from shikimic acid.

As mentioned earlier, the invention provides cosmetic compositions that make it possible to combat the effects of ageing on skin due to the process of cellular senescence, for example induced by Ultraviolet Type-B (UVB) damage, such as the appearance of wrinkles, loss of density, appearance of hyperpigmentation or loss of firmness, due to including, as a protective component against the process of cellular senescence, shikimic acid, acting through the activation of sirtuin 1 (Sirt1).

Cellular senescence is characterised by an irreversible blockage of the cell cycle and phenotypic changes in gene expression, cell functions and morphology (Kuilman T *et al.* 2010, Chung KW *et al.* 2015). For example, senescent cells exhibit increased size, increased SA-β-Gal activity, overexpression of p16 and DNA damage foci. In addition, senescent cells secrete proinflammatory cytokines which have a negative impact on the microenvironment, since they cause chronic inflammation, alter cell structure and stimulate the proliferation of malignant cells (Dilley TK *et al.* 2006, Yang G *et al.* 2006).

Cellular senescence can be induced by many stimuli, such as telomere shortening, genomic damage, strong chronic mitogenic signals or mitochondrial dysfunction (Wei W *et al.* 2018, Muñoz-Espin D *et al.* 2014). External damages such as ionising radiation, UV or H2O2 cause what is called stress-induced premature senescence (SIPS).

Both UV-induced senescence, through DNA damage and increased oxidative stress, (Dupont E *et al.* 2013), and senescence due to chronological ageing can be observed in skin (Dreesen O *et al.* 2013).

Senescent fibroblasts secrete factors, known as senescence-associated secretory phenotype or SASP. Among these factors, there are inflammatory cytokines and MMPs that degrade dermal matrix proteins such as collagen and further involve a lower expression of the type I collagen gene (Toutfaire M *et al.* 2017). Thus, the senescent fibroblast induces a disorganisation of the structure and loss of function of the dermis (Kohl E *et al.* 2011). Additionally, these molecules secreted to the matrix contribute to hyperpigmentation; therefore, the removal of senescent cells would favour skin whitening (Yoon JE *et al.* 2018).

In the case of extrinsic skin ageing, exposure to Ultraviolet UV radiation, especially UV-B (280-315 nm), has a cumulative effect that affects the exposed areas of the skin. Thus, wrinkles, pigmented lesions and depigmented lesions, loss of tone and elasticity, fragility or weakness of blood vessels are clinical signs of a photoaged skin. It is also known that exposure to UV radiation regulates the synthesis of several types of enzymes whose function is the degradation of collagen, known as matrix metalloproteinases (MMP). MMP, specifically collagenase and gelatinase, have been shown to be produced during a few hours of exposure to UV-B radiation (Fischer GJ et al., Pathophysiology of premature skin ageing induced by ultraviolet light. The New England Journal of Medicine, 1997, 337(20):1419-1428).

Since shikimic acid inhibits senescence through Sirt1, as described in this invention, it is plausible that it also inhibits senescence caused by other damage, such as other radiations or simply the passage of time (intrinsic ageing), through Sirt1, wherein shikimic acid constitutes an ingredient for general protection against ageing, whatever its cause.

In the search for anti-ageing treatments for skin, sirtuins are good targets, since they are enzymes, deacetylases, which can prevent senescence by interfering with different cellular processes. Of all the sirtuins, Sirt1 is the most widely studied.

Sirt1 activates PGC-1a by promoting mitochondrial biogenesis and the expression of antioxidant genes such as glutathione peroxidase, catalase or SOD (St-Pierre J *et al.* 2006, Scarpulla RC. 2011, Merksamer PI *et al.* 2013). Sirt1 inhibits NF-kB by decreasing inflammation (Yeung F *et al.* 2004). Sirt1 deacetylates FOXO family proteins, stimulating the transcription of antioxidant genes (Brunet A *et al.* 2004, Van der Horst A *et al.* 2004). Sirt1 activates DNA repair proteins such as XPA (Fan W 2010, Jarrett SG *et al.* 2018), BRCA1 (Lahusen TJ *et al.* 2018), Ku70 (Jeong J *et al.* 2007), GADD45a (Maeda T *et al.* 2002), KAP1 (Lin YH *et al.* 2015) and XPC (Ming M *et al.* 2010).

In the skin, Sirt1 inhibits the expression of MMP1, which is the enzyme responsible for collagen degradation (Ohguchi K *et al.* 2010) and protects fibroblasts from UVB damage (Chung KW *et al.* 2015).

In the field of the present invention, Sirt1 activators are hardly used in skin cosmetics, except for resveratrol, whose effects on ageing in general are controversial as to their mechanism of action on sirtuins (David Gems; Burnett, C. et al. Nature 477, 482-485 (2011)). In this regard, the cosmetic use of resveratrol has several disadvantages, such as its instability, its low bioavailability and its limited skin penetration (Walle T. et al., Ann NY Acad Sci. 2011; 1215: 9-15; Scalia S et al., Colloids Surf B Biointerfaces. 2015; 135: 42-9).

Given the aforementioned background, the present invention was aimed at designing cosmetic compositions that make it possible to combat the effects of ageing on skin due to the process of cellular senescence, such as the appearance of wrinkles, loss of density, appearance of hyperpigmentation or loss of firmness. Surprisingly, the present inventors have found that shikimic acid is a protective component against the process of cell senescence induced by Ultraviolet Type-B (UVB) damage acting through the activation of sirtuin 1 (Sirt1). Thus, in the present description it is shown, on the one hand, that the shikimic acid is capable of preventing UVB damage-induced cellular senescence and, on the other, that Sirt1 inhibition eliminates the protective effect of the shikimic acid compound on senescence, thereby demonstrating that shikimic acid protects against UVB-induced senescence through Sirt1.

Thus, an object of the invention is to provide cosmetic compositions that make it possible to combat the effects of ageing on skin due to the process of cellular senescence, for example induced by Ultraviolet Type-B (UVB) damage, which include, as a protective component against the process of cellular senescence, shikimic acid.

Another object of the invention is the use of shikimic acid for treating the ageing of skin, through protection against the process of cellular senescence by means of sirtuin 1.

According to the first object, cosmetic compositions are provided which include, as a protective component against the process of cellular senescence induced by Ultraviolet Type-B (UVB) damage, shikimic acid.

In preferred embodiments, shikimic acid is present in these cosmetic compositions in a proportion of 0.5 to 5.0 % by weight with respect to the total weight of the cosmetic composition.

The cosmetic composition of the invention including shikimic acid is not limited in its form of presentation, and can be constituted as a cream, a lotion and the like. To this end, the composition of the invention may include, in addition to shikimic acid, common components for this type of formulations, such as water, chelators, stabilisers, preservatives, antioxidants, emollients, emulsifiers, silicones, pigments, opacifiers, humectants, fragrance, viscosity agents, buffers, solubilisers, etc.

Examples of chelators for use in this composition are selected from the group consisting of edetates or phytic acid and mixtures thereof, and are preferably used in a proportion of 0.1 to 5% by weight with respect to the weight of the cosmetic composition.

Examples of preservatives for use in this composition are selected from the group consisting of enphenoxyethanol, ethylhexyl glycerine, sodium benzoate, potassium sorbate, 1,6-hexylene glycol, capryloyl glycol mixtures thereof and, if present, preferably used in a proportion 0.5 % to 1 % by weight with respect to the weight of the cosmetic composition.

Examples of humectants for use in this composition are selected from the group consisting of glycerine, butylene glycol, propylene glycol, propanediol, pentylene glycol, ethylhexyl glycerine, polyethylene glycol ether glycerine and mixtures thereof and, if present, preferably used in a proportion of 0.5 % to 15 % by weight with respect to the weight of the cosmetic composition.

Examples of emulsifiers for use in this composition are selected from the group consisting of glyceryl stearate, polyethylene glycol stearate, polysorbate 20, polysorbate 80, lysolecithin, sorbitan oleate, stearyl alcohol, sodium lauryl sulfate, polyethylene glycol C12-C20 fatty acid esters, PEG-10 dimethicone, disteardimonium hectorite, cetyl PEG/PPG-10/1 dimethicone and sodium cetearyl sulfate and mixtures thereof and, if present, are preferably used in a proportion of 0.5 % to 10 % by weight with respect to the weight of the cosmetic composition.

Examples of viscosity agents for use in this composition are selected from the group consisting of xanthan gum, guar gum, sclerotium gum, carbomers, celluloses, polyacrylates, carrageenates, magnesium chloride, hydroxystearic acid, lecithin and mixtures thereof and, if present, are preferably used in a proportion of 0.1 % to 5 % by weight with respect to the weight of the cosmetic composition.

Examples of emollients for use in this composition are selected from the group consisting of glycerol esters of C8-C12 fatty acids, paraffin, dibutyl adipate, dicaprilyl carbonate, isodecyl neopentanoate, n-undecane mixtures: n-tridecane (C11/C13), triheptanoin, isononyl isononanoate, alkyl benzoate (C12-C15), natural oils, butters, tridecyl stearate, neopentyl glycol diacaprylate/dicaprate, tridecyl trimethylate, squalene, isohexadecane, coconut caprate/caprylate and mixtures thereof and, if present, are preferably used in a proportion of 1.0 % to 40 % by weight with respect to the weight of the cosmetic composition.

Examples of silicones for use in this composition are selected from the group consisting of cyclopentasiloxane, dimethicone, cyclohexasilane, silicone elastomers, simethicone, caprilylmethicone, HDI/trimethylolhexyllactone crosspolymer and silica, cetearyl methicone and mixtures thereof and, if present, are preferably used in a proportion of 0.5 % to 15 % by weight with respect to the weight of the cosmetic composition.

Examples of antioxidants for use in this composition are selected from the group consisting of tocopherol, vitamin E acetate, butylhydroxytoulene, butylhydroxyanisole, pentaerythrityltetra-di-t-butylhydroxyhydrocinamate, ginger root extract, benzotriazolylbutylphenylsulfonate sodium, PEG-8, palmitate ascorbyl, citric acid and mixtures thereof and, if present, are preferably used in a proportion of 0.05 % to 2 % by weight with respect to the weight of the cosmetic composition.

Examples of buffers for use in this composition are selected from the group consisting of sodium hydroxide, triethanolamine, tromethamine and mixtures thereof, and, if present, are preferably used in a proportion of 0.5 % to 2 % by weight with respect to the weight of the cosmetic composition.

Examples of opacifiers for use in this composition are selected from the group consisting of polymethylsilylsesquixane, acrylate-styrene copolymers and mixtures thereof and, if present, are preferably used in a proportion of 0.5 % to 5 % by weight with respect to the weight of the cosmetic composition.

Examples of solubilisers for use in this composition are selected from the group consisting of PEG-40 hydrogenated castor oil, PEG-8, TRIDECETH-9, POLISORBATE 20, PPG-26-BUTETH-26 and mixtures thereof and, if present, are preferably used in a proportion of 0.5 % to 2.5 % by weight with respect to the weight of the cosmetic composition.

Thus, in a preferred embodiment, the cosmetic composition of the invention is a base cream with the following components and in the following proportions:

**Table 1**

| **Components** | **% by weight** |
|---|---|
| water | up to 100 |
| humectants | 2-15 |
| chelators | 0.1 |
| preservatives | 0.5-1 |
| viscosity agents | 0.1-2 |
| emulsifiers | 0.5-7 |
| emollients | 2-15 |
| silicones | 0.5-10 |
| antioxidants | 0.5-2 |
| shikimic acid | 0.5-5.0 |
| fragrance | 0.5-3 |

In another preferred embodiment, the cosmetic composition of the invention is a lotion with the following components and in the following proportions:

**Table 2**

| **Components** | **% by weight** |
|---|---|
| water | up to 100 |
| humectants | 0.5-10 |
| preservatives | 0.5-1 |
| denatured alcohol | 30-45 |
| buffer | 0.5-2 |
| shikimic acid | 0.5-5.0 |

In yet another preferred embodiment, the cosmetic composition of the invention is a redensifying cream with the following components and in the following proportions:

**Table 3**

| **Components** | **% by weight** |
|---|---|
| water | up to 100 |
| chelators | 0.1-2 |
| humectants | 0.5-10 |
| preservatives | 0.5-1 |
| emollients | 1-40 |
| emulsifiers | 0.5-7 |
| viscosity agents | 0.2-3 |
| antioxidants | 0.05-2 |
| fragrance | 0.2-2 |
| shikimic acid | 0.5-5.0 |

In another additional preferred embodiment, the cosmetic composition of the invention is a whitening cream with the following components and in the following proportions:

**Table 4**

| **Components** | **% by weight** |
|---|---|
| water | up to 100 |
| humectants | 0.5-10 |
| preservatives | 0.5-1 |
| fragrance | 0.2-2 |
| solubilisers | 0.5-2.5 |
| dye | 0.01-2 |
| buffer | 0.5-2 |
| shikimic acid | 0.5-5.0 |

In yet another additional preferred embodiment, the cosmetic composition of the invention is an anti-wrinkle cream with the following components and in the following proportions:

**Table 5**

| **Components** | **% by weight** |
|---|---|
| water | up to 100 |
| viscosity agents | 0.5-3 |
| humectants | 0.5-10 |
| preservatives | 0.5-1 |
| silicones | 0.5-15 |
| emollients | 1-40 |
| emulsifiers | 0.5-10 |
| opacifiers | 0.5-5 |
| antioxidants | 0.05-2 |
| pigments | 0.2-2 |
| fragrance | 0.2-2 |
| shikimic acid | 0.5-5.0 |

In another additional preferred embodiment, the cosmetic composition of the invention is a firming cream with the following components and in the following proportions:

**Table 6**

| **Components** | **% by weight** |
|---|---|
| water | up to 100 |
| chelators | 0.1-0.5 |
| humectants | 0.5-10 |
| preservatives | 0.5-1 |
| viscosity agents | 0.2-5 |
| opacifiers | 0.5-2 |
| antioxidants | 0.05-2 |
| fragrance | 0.2-2 |
| shikimic acid | 0.5-5.0 |

According to another object of the invention, this refers to the use of shikimic acid in a cosmetic composition as a protector against the process of cellular senescence induced by Ultraviolet type-B (UVB) damage.

In order to verify the action of the shikimic acid as a protector against the process of cellular senescence induced by Ultraviolet Type-B (UVB) damage, the shikimic acid was tested in a model of UVB-induced senescence in human dermal fibroblasts. In this model, two days after being plated, the cells are irradiated with UVB. After three days, the cells are re-irradiated with UVB. Two days after the second irradiation, the cells are processed for analysis. The shikimic acid is incubated with the cells from 24 h before the first irradiation until the day they are analysed.

To assess whether cells are senescent or not, two markers are used. On the one hand, β-galactosidase staining is carried out, which stains the senescent cells blue, since they have an abnormal lysosomal activity at pH 6. Furthermore, the expression of the *HAS2* gene is quantified by means of the qPCR technique. This gene corresponds to hyaluronate synthase 2 and it has been described that the expression of this gene is inhibited in senescent cells (J Invest Dermatol. 2015 Feb; 135(2): 369-377. doi: 10.1038/jid.2014.422. Epub 2014 Sep 29, "miR-23a-3p causes cellular senescence by targeting hyaluronan synthase 2: possible implication for skin ageing", Rock K. at al.)

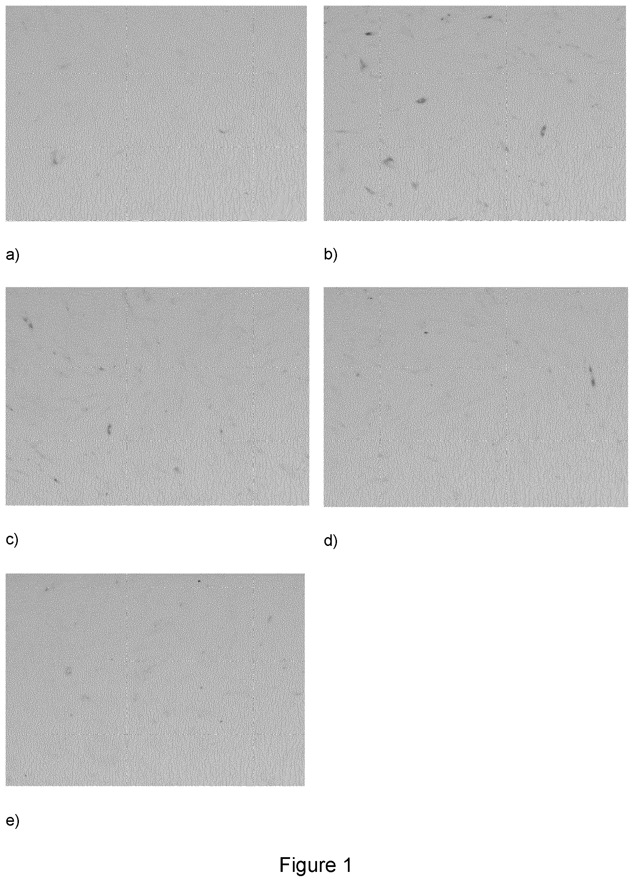

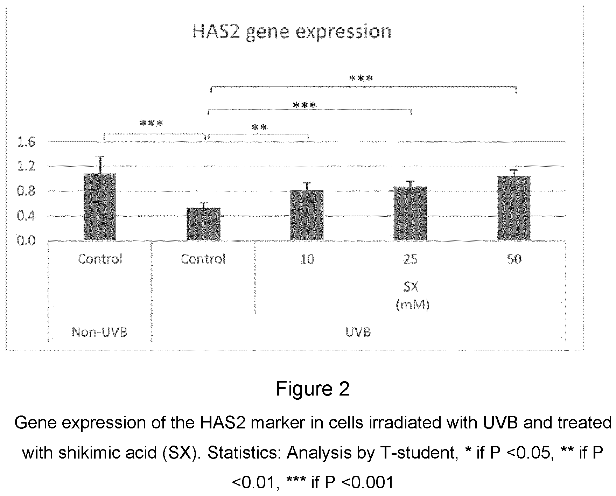

The results of the staining obtained are shown in Figure 1, which are micrographs of the β-Galactosidase staining, and Figure 2, which shows the graph comparing the HAS2 gene expression, under different conditions: a) non-UVB-irradiated and b) UVB-irradiated, c) UVB-irradiated + treated with 10 mM shikimic acid, d) UVB-irradiated + treated with 25 mM shikimic acid, and e) UVB-irradiated + treated with 50 mM shikimic acid.

As can be observed in Figure 1, an increase in senescent cells occurs after irradiation with UVB and, however, when the cells are irradiated and treated with shikimic acid, this increase in senescence does not occur.

Regarding the HAS2 expression, in Figure 2 it is observed that its expression decreases in UVB-irradiated cells, but in cells irradiated with UVB and treated with shikimic acid (SX), this decrease is reversed in gene expression.

Once these results have been obtained, in order to check if the effect of the shikimic acid is produced through sirtuins, different specific inhibitors thereof are used: EX-527 (inhibits Sirt1 and, to a lesser extent, SIRT6), AGK2 (inhibits SIRT2) and OSS128167 (inhibits SIRT6). Therefore, the effect of inhibitors on the protective role of shikimic acid is tested in the same UVB-induced senescence model.

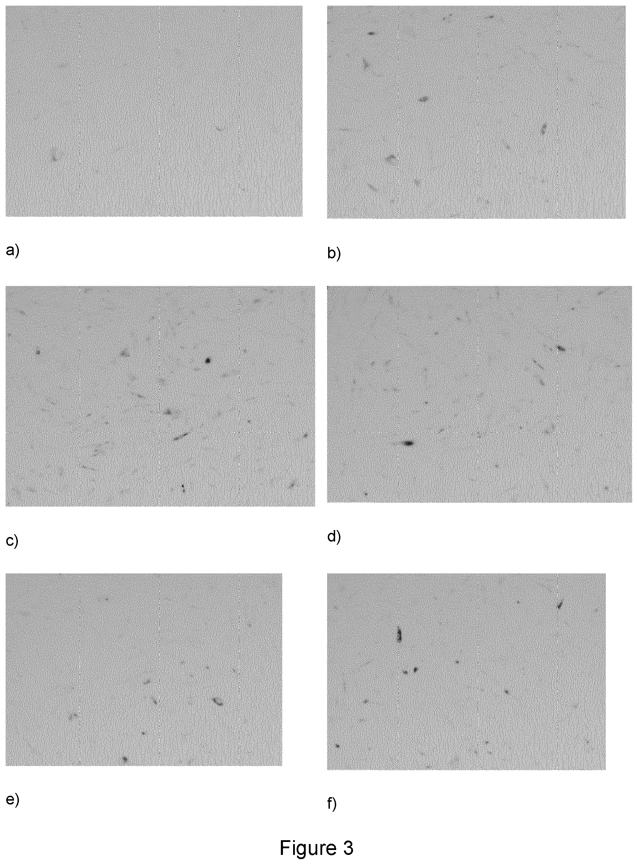

Figure 3 shows photos of β-galactosidase staining of cells irradiated with UVB and treated with shikimic acid (SX) + inhibitor EX-5271µM: a) non-UVB-irradiated (control) and b) UVB-irradiated (control), c) UVB-irradiated + treated with 10 mM shikimic acid + EX5271µM, d) UVB-irradiated + treated with 25 mM shikimic acid + EX5271µM, e) UVB-irradiated + treated with 50 mM shikimic acid + EX5271µM and f) UVB-irradiated + EX5271µM (without shikimic acid).

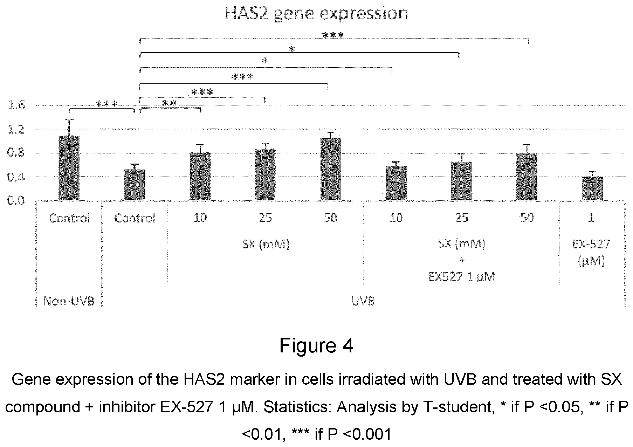

Figure 4 shows graphs corresponding to the HAS2 gene expression of non-irradiated cells (control), UVB-irradiated cells (control), treated with shikimic acid (SX) and with shikimic acid and the inhibitor EX5271µM.

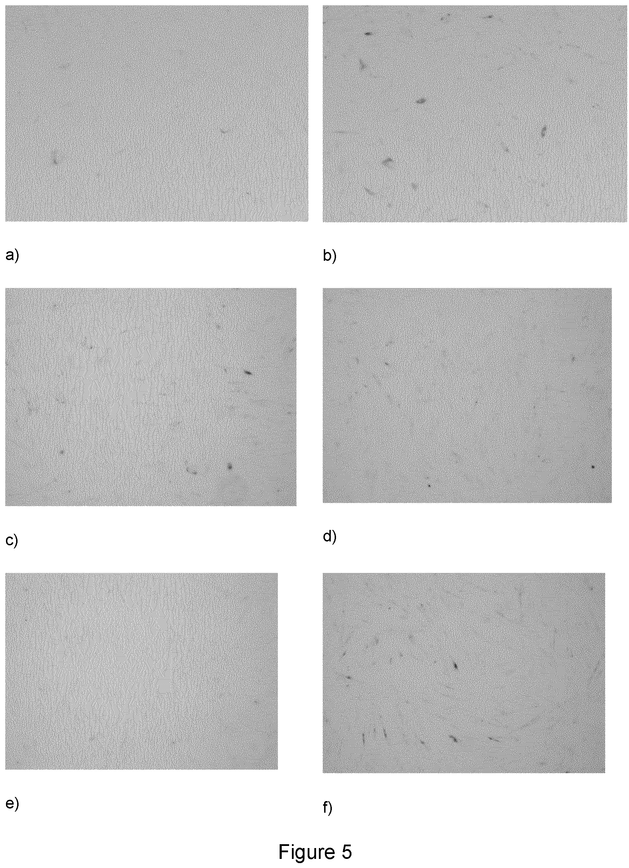

Figure 5 shows photos of β-galactosidase staining of cells irradiated with UVB and treated with shikimic acid (SX) + AGPK22µM inhibitor: a) non-UVB-irradiated (control) and b) UVB-irradiated (control), c) UVB-irradiated + treated with 10 mM shikimic acid + AGK22µM, d) UVB-irradiated + treated with 25 mM shikimic acid + AGK22µM, e) UVB-irradiated + treated with 50 mM shikimic acid + AGPK22µM and f) UVB-irradiated + AGPK22µM (without shikimic acid).

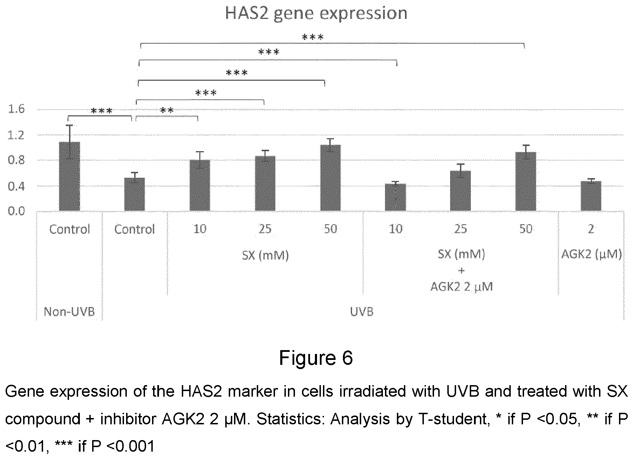

Figure 6 shows graphs corresponding to the HAS2 gene expression of non-irradiated (control) cells, UVB-irradiated cells (control), treated with shikimic acid (SX) and with shikimic acid and the AGPK22µM inhibitor.

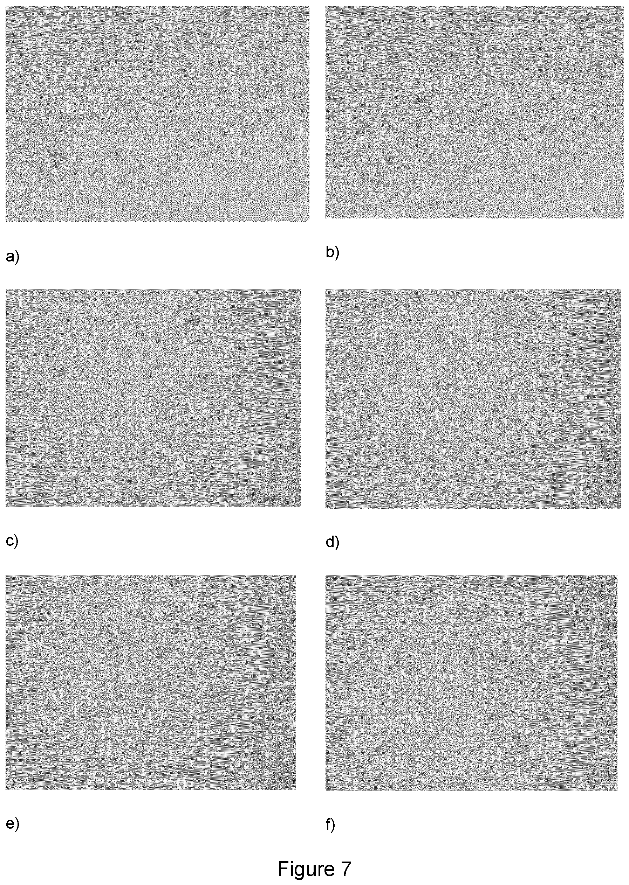

Figure 7 shows photos of β-galactosidase staining of cells irradiated with UVB and treated with shikimic acid (SX) + inhibitor OSS128167 200 µM: a) non-UVB-irradiated (control) and b) UVB-irradiated (control), c) UVB-irradiated + treated with 10 mM shikimic acid + OSS128167 200 µM, d) UVB-irradiated + treated with 25 mM shikimic acid + OSS128167 200 µM, e) UVB-irradiated + treated with 50 mM + OSS128167 200 µM and f) UVB-irradiated + OSS128167 200 µM (without shikimic acid).

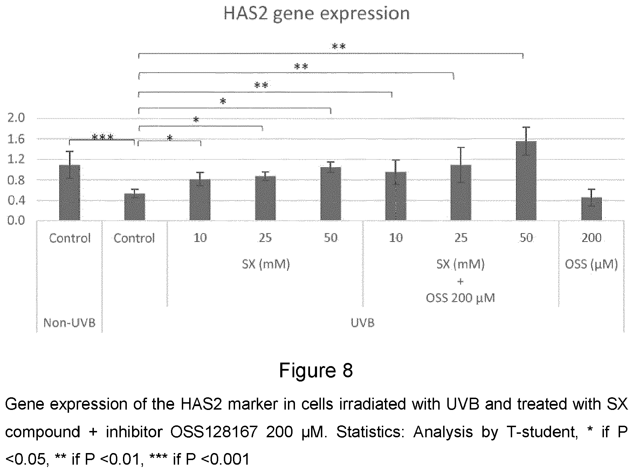

Figure 8 shows graphs corresponding to the HAS2 gene expression of non-irradiated cells (control), UVB-irradiated cells (control), treated with shikimic acid (SX) and with shikimic acid and the inhibitor OSS128167 200 µM.

According to these results, it is observed that the selective inhibitor of Sirt1 EX-527 is capable of reversing the effect of shikimic acid on senescence, since more senescent cells appear in the cells treated with this compound (SX) + inhibitor EX-527 than when treated only with the compound SX. In addition, gene expression of HAS2 is reversed to a lesser extent in cells treated with shikimic acid (SX) + inhibitor EX-527 compared to cells treated only with SX.

Furthermore, the other two inhibitors tested (for SIRT2 and SIRT6) do not reverse the protective effect of shikimic acid on senescence, since a similar result is obtained by incubating the cells with shikimic acid (SX) + inhibitor AGK2 or OSS128167 compared to the incubation of the cells with shikimic acid alone.

Thus, it can be concluded that the protective effect of the compound SX on UVB damage-induced senescence occurs specifically through Sirt1.

In parallel to the study of the effect of shikimic acid on UVB damage-induced senescence in cells, other compounds included in anti-ageing products were tested for use in cosmetics, idebenone and lipoic acid through the staining assay described above.

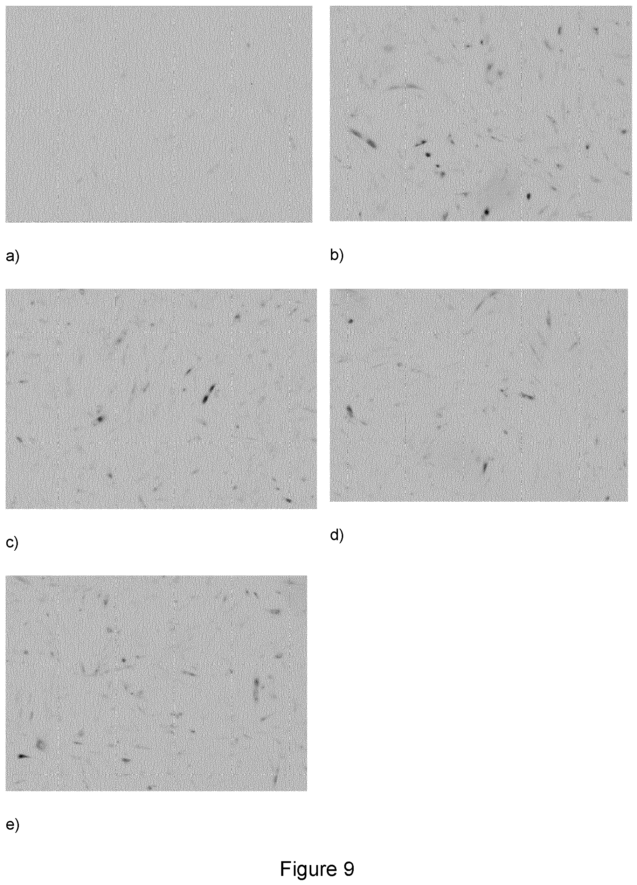

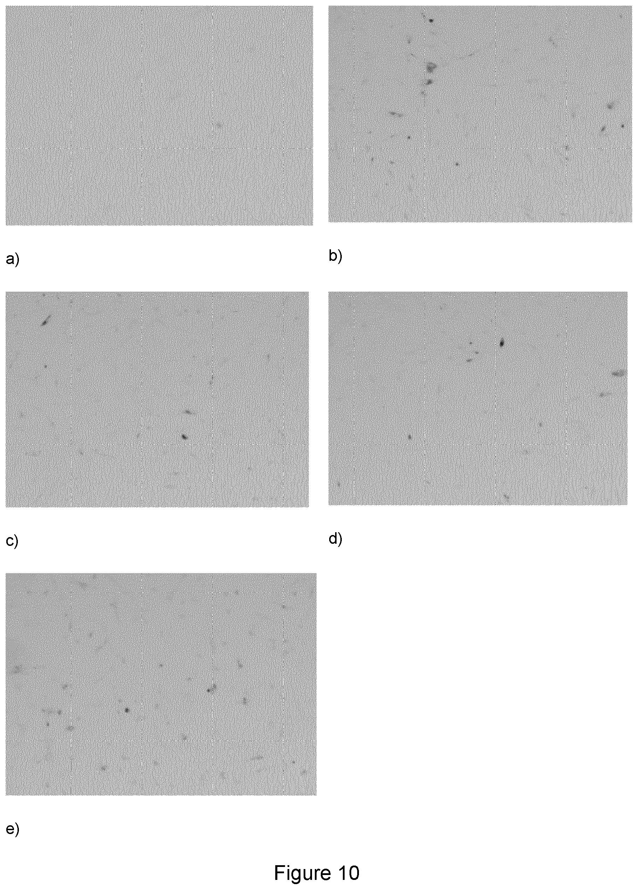

The results of these comparative tests are shown in Figures 9 and 10. Figure 9 shows staining with β-Galactosidase, treated with idebenone, a) non-UVB-irradiated and b) UVB-irradiated, c) UVB-irradiated + treated with 2.5µM idebenone, d) UVB-irradiated + treated with 5µM idebenone and) UVB-irradiated + treated with 10µM idebenone.

The results obtained with the lipoic acid treatment are shown in Figure 10, a) non-UVB-irradiated and b) UVB-irradiated, c) UVB-irradiated + treated with 50 µM lipoic acid, d) UVB-irradiated + 100µM lipoic acid and e) UVB-irradiated + 200µM lipoic acid.

As derived from Figures 9 and 10, neither of these two comparative compounds is capable of protecting against UVB damage-induced senescence in this model. Therefore, shikimic acid is more effective than other compounds such as idebenone or lipoic acid in protecting against the photoageing process in human dermal fibroblasts.

In conclusion, the composition of the invention represents an innovation with respect to other anti-ageing products on the market, since there are not many Sirt1 activators used in skin cosmetics. Shikimic acid is capable of protecting against UVB damage-induced senescence, a process that occurs in the skin with age. Thus, it protects against the harmful effect of senescent cells on tissues, which cells create an inflammatory microenvironment and induce the degradation of matrix proteins such as collagen. In addition, this protection by shikimic acid is carried out through the action of Sirt1, a protein that, through epigenetic mechanisms, is capable of regulating various cellular processes (DNA repair, metabolism or autophagy, inter alia) and activating cellular stress response, protecting cells from external damage.

### Examples

In the following example, indicated by way of illustration and not limitation of the invention, a cosmetic composition of the invention is shown as a base cream.

### Example 1: Cosmetic composition in the form of a treatment base cream

| **Components** | **% by weight** |
|---|---|
| water | qsp 100 |
| glycerine | 4.0 |
| EDTA-disodium | 0.1 |
| phenoxyethanol-ethylhexyl glycerine | 1.0 |
| sclerotium gum | 0.8 |
| cetearyl alcohol, sodium lauryl sulfate, sodium cetearyl sulfate | 3.5 |
| sodium hydroxide | 0.9 |
| tridecyl stearate, tridecylohexacaprylate trimethylate/dipentaerythrityl hexacaprate | 2.0 |
| PEG-6 esters of apricot kernel oil | 2.0 |
| alkyl benzoate (C12-15) | 4.0 |
| caprylic/capric triglycerides | 3.0 |
| cyclopentasiloxane | 2.0 |
| dimethicone | 2.0 |
| simethicone | 0.05 |
| pentaerythrityltetra-di-t-butylhydroxyhydrocinamate | 0.1 |
| PEG-8 C12-20 acid esters | 2.5 |
| shikimic acid | 1.0 |
| styrene/acrylate copolymer | 0.5 |
| fragrance | 0.3 |

## Claims

1. Cosmetic compositions **characterised in that** they include a protective component against the process of cellular senescence selected from shikimic acid, acting through the activation of sirtuin 1.

2. The cosmetic compositions, according to claim 1, **characterised in that** shikimic acid is present in a proportion of 0.05 % to 5.0 % by weight with respect to the total weight of the cosmetic composition.

3. The cosmetic compositions, according to claim 1 or 2, **characterised in that** they also include water, chelators, stabilisers, preservatives, antioxidants, emollients, emulsifiers, silicones, pigments, opacifiers, humectants, fragrance, viscosity agents, buffers and solubilisers.

4. The cosmetic compositions, according to claim 3, **characterised in that** the chelators are selected from the group consisting of edetates or phytic acid and mixtures thereof and are used in a proportion of 0.1 % to 5 % by weight with respect to the weight of the cosmetic composition.

5. The cosmetic compositions, according to claim 3, **characterised in that** the preservatives are selected from the group consisting of enphenoxyethanol, ethylhexyl glycerine, sodium benzoate, potassium sorbate, 1,6-hexylene glycol, capryloyl glycol mixtures thereof and are used in a proportion 0.5 % to 1 % by weight with respect to the weight of the cosmetic composition.

6. The cosmetic compositions, according to claim 3, **characterised in that** the humectants are selected from the group consisting of glycerine, butylene glycol, propylene glycol, propanediol, pentylene glycol, ethylhexyl glycerine, polyethylene glycol ether glycerine and mixtures thereof, and are used in a proportion of 0.5 % to 15 % by weight with respect to the weight of the cosmetic composition.

7. The cosmetic compositions, according to claim 3, **characterised in that** the emulsifiers are selected from the group consisting of glyceryl stearate, polyethylene glycol stearate, polysorbate 20, polysorbate 80, lysolecithin, sorbitan oleate, stearyl alcohol, sodium lauryl sulfate, polyethylene glycol C12 fatty acid esters -C20, PEG-10 dimethicone, disteardimonium hectorite, cetyl PEG/PPG-10/1 dimethicone and sodium cetearyl sulfate and mixtures thereof, and are used in a proportion of 0.5 % to 10 % by weight with respect to the weight of the cosmetic composition.

8. The cosmetic compositions, according to claim 3, **characterised in that** the viscosity agents are selected from the group consisting of xanthan gum, guar gum, sclerotium gum, carbomers, celluloses, polyacrylates, carrageenates, magnesium chloride, hydroxystearic acid, lecithin and mixtures thereof, and are used in a proportion of 0.1 % to 5 % by weight with respect to the weight of the cosmetic composition.

9. The cosmetic compositions, according to claim 3, **characterised in that** the emollients are selected from the group consisting of glycerol esters of C8-C12 fatty acids, paraffin, dibutyl adipate, dicaprilyl carbonate, isodecyl neopentanoate, n-undecane mixtures: n-tridecane (C11/C13), triheptanoin, isononyl isononanoate, alkyl benzoate (C12-C15), natural oils, butters, tridecyl stearate, neopentyl glycol diacaprylate/dicaprate, tridecyl trimethylate, squalene, isohexadecane, coconut caprate/capylate and mixtures thereof, and are used in a proportion of 1.0 % to 40 % by weight with respect to the weight of the cosmetic composition.

10. The cosmetic compositions, according to claim 3, **characterised in that** the silicones are selected from the group consisting of cyclopentasiloxane, dimethicone, cyclohexasilane, silicone elastomers, simethicone, caprilylmethicone, HDI/trimethylolhexyllactone crosspolymer and silica, cetearyl methicone and mixtures thereof, and are used in a proportion of 0.5 % to 15 % by weight with respect to the weight of the cosmetic composition.

11. The cosmetic compositions, according to claim 3, **characterised in that** the antioxidants are selected from the group consisting of tocopherol, vitamin E acetate, butylhydroxytoulene, butylhydroxyanisole, pentaerythrityltetra-di-t-butylhydroxyhydrocinamate, ginger root extract, benzotriazolylbutylphenylsulfonate sodium, PEG-8, palmitate ascorbyl, citric acid and mixtures thereof, and are used in a proportion of 0.05 % to 2 % by weight with respect to the weight of the cosmetic composition.

12. The cosmetic compositions, according to claim 3, **characterised in that** the buffers are selected from the group consisting of sodium hydroxide, triethanolamine, tromethamine and mixtures thereof, and are used in a proportion of 0.5 % to 2 % by weight with respect to the weight of the cosmetic composition.

13. The cosmetic compositions, according to claim 3, **characterised in that** the opacifiers are selected from the group consisting of polymethylsilylsquixane, acrylate-styrene copolymers and mixtures thereof, and are used in a proportion of 0.5 % to 5 % by weight with respect to weight of the cosmetic composition.

14. The cosmetic compositions, according to claim 3, **characterised in that** the solubilisers are selected from the group consisting of PEG-40 hydrogenated castor oil, PEG-8, TRIDECETH-9, POLISORBATE 20, PPG-26-BUTETH-26 and mixtures thereof, and are used in a proportion of 0.5 % to 2.5 % by weight with respect to the weight of the cosmetic composition.

15. Use of shikimic acid in a cosmetic composition as a protector against the process of cellular senescence.
